(19) Europäisches Patentamt
European Patent Office
Office européen des brevets

(11) Veröffentlichungsnummer : **0 412 391 B1**

(12) # EUROPÄISCHE PATENTSCHRIFT

(45) Veröffentlichungstag der Patentschrift : **29.09.93 Patentblatt 93/39**

(51) Int. Cl.[5] : **C07C 327/46,** A01N 53/00

(21) Anmeldenummer : **90114590.4**

(22) Anmeldetag : **30.07.90**

(54) **Cyclopropanthiocarbonsäureamide.**

(30) Priorität : **10.08.89 DE 3926468**

(43) Veröffentlichungstag der Anmeldung : **13.02.91 Patentblatt 91/07**

(45) Bekanntmachung des Hinweises auf die Patenterteilung : **29.09.93 Patentblatt 93/39**

(84) Benannte Vertragsstaaten : **AT BE CH DE ES FR GB IT LI NL SE**

(56) Entgegenhaltungen :
**EP-A- 0 258 733**
**EP-A- 0 350 688**
**PATENT ABSTRACTS OF JAPAN, vol. 1, no. 114, 4273 C 77, 30. September 1977; & JP-A-52 128220**
**CESARE FERRI: "REAKTIONEN DER ORGANISCHEN SYNTHESE", 1978, GEORG THIEME VERLAG, STUTTGART**
**HOUBEN WEYL: "METHODEN DER ORGANISCHEN CHEMIE", 1965, GEORG THIEME VERLAG, STUTTGART BAND VI/3**

(56) Entgegenhaltungen :
**COMPTES RENDUS DES SEANCES DE L'ACADEMIE DES SCIENCES, vol. 274, 7. Februar 1972, MONTREUIL, FR, Seiten 642 - 645; J.-M. BEINER ET AL.: "PREPARATION D'ACIDES DITHIOCARBOXYLIQUES SATURES ET DE LEUR ESTERS"**

(73) Patentinhaber : **BASF Aktiengesellschaft**
**Carl-Bosch-Strasse 38**
**D-67063 Ludwigshafen (DE)**

(72) Erfinder : **Kardorff, Uwe, Dr.**
**D-3,4**
**D-6800 Mannheim 1 (DE)**
Erfinder : **Leyendecker, Joachim, Dr.**
**Stahlbuehlring 79**
**D-6802 Ladenburg (DE)**
Erfinder : **Neubauer, Hans-Juergen, Dr.**
**Mozartstrasse 6**
**D-4400 Muenster (DE)**
Erfinder : **Kuenast, Christoph, Dr.**
**Salierstrasse 2**
**D-6701 Otterstadt (DE)**
Erfinder : **Hofmeister, Peter, Dr.**
**Bernard-Humblot-Strasse 12**
**D-6730 Neustadt (DE)**
Erfinder : **Krieg, Wolfgang, Dr.**
**Saarstrasse 17**
**D-6721 Weingarten (DE)**

EP 0 412 391 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue Cyclopropanthiocarbonsäureamide der allgemeinen Formel I

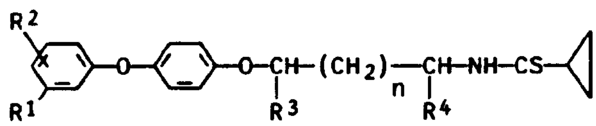

(I)

in der die Variablen folgende Bedeutung haben:

$R^1$, $R^2$ Wasserstoff, Cyano, Nitro, Halogen, $C_1$-$C_4$-Alkyl oder $C_1$-$C_4$-Alkoxy, wobei die Alkyl- und Alkoxyreste partiell oder vollständig halogeniert sein können;

$R^3$, $R^4$ Wasserstoff oder $C_1$-$C_4$-Alkyl;

n 0 oder 1.

Außerdem betrifft die Erfindung ein Verfahren zur Herstellung dieser Verbindungen, ihre Verwendung zur Schädlingsbekämpfung sowie Mittel, welche diese Verbindungen als wirksame Substanzen enthalten.

Aus der DE-A 36 28 082 sind N-[2-(4-Phenoxy-phenoxy)ethyl]-cyclopropancarbonsäureamide als pestizide Verbindungen bekannt.

Die Wirkung dieser bekannten Pestizide auf die Schädlinge sowie die Dauer ihrer Wirkung vermag jedoch nur bedingt zu befriedigen. Daher lagen der Erfindung neue Schädlingsbekämpfungsmittel als Aufgabe zugrunde, mit denen sich die Schädlinge besser als bisher bekämpfen lassen.

Demgemäß wurden die eingangs definierten Cyclopropanthiocarbonsäureamide der allgemeinen Formel I gefunden.

Im einzelnen haben die Substituenten in den erfindungsgemäßen Verbindungen I die folgende Bedeutung:

$R^1$

- Wasserstoff, Cyano, Nitro;
- Halogen, darunter bevorzugt Fluor, Chlor und Brom;
- unverzweigtes oder verzweigtes $C_1$-$C_4$-Alkyl wie Methyl, Ethyl, Propyl, Isopropyl, Butyl, Isobutyl, sec.-Butyl oder tert.-Butyl, bevorzugt Methyl, Ethyl, Isopropyl, Isobutyl und sec.-Butyl;
- unverzweigtes oder verzweigtes $C_1$-$C_4$-Alkoxy wie vorzugsweise Methoxy, Ethoxy und Isopropoxy;
- partiell oder vollständig halogeniertes $C_1$-$C_4$-Alkyl, vorzugsweise unverzweigtes oder verzweigtes $C_1$-$C_4$-Fluor- und $C_1$-$C_4$-Chloralkyl wie Fluormethyl, Difluormethyl, Trifluormethyl, 2,2,2-Trifluorethyl, Pentafluorethyl, Trichlormethyl und 2,2,2-Trichlorethyl;
- partiell oder vollständig halogeniertes $C_1$-$C_4$-Alkoxy, vorzugsweise unverzweigtes oder verzweigtes $C_1$-$C_4$-Fluor- und $C_1$-$C_4$-Chloralkoxy wie Trifluormethoxy, 1,1,2,2-Tetrafluorethoxy, Pentafluorethoxy und Trichlormethoxy;

$R^2$ Für diesen Substituenten, der bevorzugt in 4-Stellung des Phenylrings steht, gilt das gleiche wie für den Substituenten $R^1$;

$R^3$, $R^4$ Wasserstoff, sowie daneben Methyl, Ethyl, Propyl, Isopropyl, Butyl, Isobutyl, sec.-Butyl und tert.-Butyl, bevorzugt Methyl und Ethyl.

Besonders gut geeignet sind Verbindungen I, in denen $R^3$ und $R^4$ Wasserstoff bedeuten und n = 0 ist. Bevorzugte Verbindungen I sind den Beispielen zu entnehmen.

Die erfindungsgemäßen Verbindungen I können nach an sich bekannten Verfahren der Thiocarbonsäureamidsynthese dargestellt werden (vgl. C. Ferri, Reaktionen der organischen Synthese, Georg Thieme Verlag, Stuttgart 1978, S. 549 ff.), z.B. durch Schwefelung der entsprechenden Cyclopropancarboxamide mit $P_4S_{10}$ oder mit Lawesson-Reagenz (2,4-Bis-(4-methoxyphenyl)-2,4-dithioxo-1,3,2,4-dithiadiphosphetan).

Besonders vorteilhaft erhält man die Verbindungen I durch Umsetzung von 4-Phenoxyphenoxyalkylaminen II

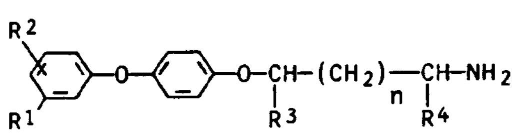

(II)

mit einem Cyclopropandithiocarbonsäureester III

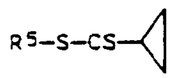

(III)

vorzugsweise einem $C_1$-$C_6$-Alkylester wie vor allem dem Methylester.

Bevorzugt setzt man die Ausgangsverbindungen II und III im stöchiometrischen Verhältnis ein, jedoch kann sich in manchen Fällen auch ein Überschuß der einen oder anderen Komponente, etwa bis zu 10 %, empfehlen.

Die Umsetzung verläuft gewöhnlich oberhalb von (-30)°C mit ausreichender Geschwindigkeit. Im allgemeinen liegt die Temperatur bei (-30) bis 130°C, insbesondere bei (-10) bis 80°C, bevorzugt 0 bis 50°C.

Sie wird zweckmäßigerweise in einem Lösungs- oder Verdünnungsmittel bei Normaldruck durchgeführt. Geringerer oder höherer Druck ist möglich, bringt im allgemeinen aber keine Vorteile.

Als Lösungs- oder Verdünnungsmittel eignen sich aliphatische und aromatische, gegebenenfalls chlorierte Kohlenwasserstoffe wie Petrolether, n-Pentan, n-Hexan, Hexan-Isomerengemische, Benzol, Toluol, Xylol, Benzin, Dichlormethan, Chloroform, Tetrachlorkohlenstoff, 1,2-Dichlorethan und Chlorbenzol; Ether wie Diethyl- und Di-n-butylether, Methyl-tert.-butylether, Tetrahydrofuran und Dioxan; Ester wie Essigsäureethylester; Ketone wie Aceton, Methylethylketon und Methylisopropylketon; Nitrile wie Aceto- und Propionitril; Alkohole wie Methanol, Ethanol, n-Propanol und Isopropanol und aprotische dipolare Lösungsmittel wie Dimethylformamid, Dimethylsulfoxid und Pyridin. Auch Gemische dieser Stoffe können als Lösungs- und Verdünnungsmittel verwendet werden.

Die zur Herstellung der Verbindungen I benötigten 4-Phenoxyphenoxyalkylamine II sind zum Teil aus Houben/Weyl, Bd. VI/3, Methoden der organischen Chemie, Thieme Verlag, 1965, S. 85 ff., bekannt oder können nach den dort beschriebenen Methoden hergestellt werden.

Die außerdem benötigten Cyclopropandithiocarbonsäureester III sind aus C.R. Acad. Sci., Ser. C, 274, 642 (1972) bekannt oder können nach der dort beschriebenen Methode hergestellt werden. Man erhält sie z.B. in einer Grignard-Reaktion durch Umsetzung von Cyclopropylbromid, Magnesium und Schwefelkohlenstoff und weiterer Umsetzung des entstandenen Dithiocarbonsäure salzes mit einem Alkyljodid, vorzugsweise Methyljodid.

Die neuen Verbindungen I fallen teilweise in Form farbloser oder schwach bräunlich gefärbter Öle an, die sich durch längeres Erwärmen unter vermindertem Druck auf mäßig erhöhte Temperaturen ("Andestillieren") von den letzten flüchtigen Anteilen befreien und auf diese Weise reinigen lassen. Erhält man die Verbindungen der Formel I in kristalliner Form, so kann man sie durch Umkristallisation reinigen.

Die erfindungsgemäßen Verbindungen I können ein oder mehrere Asymmetriezentren enthalten. Sie wirken als Racemate, wie sie bei den meisten Herstellungsverfahren anfallen, können gewünschtenfalls aber auch nach den hierfür üblichen Methoden, z.B. durch Chromatographie an einem optisch aktiven Adsorptionsmittel, in die reinen Isomeren aufgetrennt werden.

Im Gegensatz zu dem meisten bisher bekannten Schädlingsbekämpfungsmitteln, die als Kontakt- oder Fraßgifte die Tiere töten, lähmen oder vertreiben, greifen die Verbindungen der Formel I in das Hormonsystem des tierischen Organismus ein. Bei Insekten wird beispielsweise die Umwandlung zur Imago, die Ablage von entwicklungsfähigen Eiern und die Entwicklung von abgelegten, normalen Eiern gestört und dadurch die Generationsfolge unterbrochen. Für Wirbeltiere sind die erfindungsgemäßen Mittel praktisch ungiftig.

Herstellungsbeispiele

[N-[4-(3-fluor-phenoxy)-phenoxy]ethyl]-cyclopropanthiocarbonsäureamid

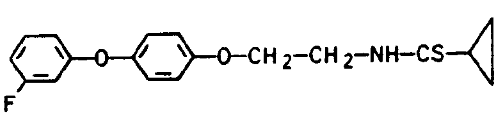

Eine Lösung aus 2,4 g (9,7 mmol) [4-(3-fluorphenoxy)-phenoxy]-ethylamin, 1,3 g (9,7 mmol) cyclopropandithiocarbonsäuremethylester und 40 ml Toluol wurde 14 Stunden lang bei 20°C gerührt, wonach die leichtflüchtigen Substanzen unter vermindertem Druck abdestilliert wurden. Der Rückstand wurde aus Ethylacetat/n-Hexan umkristallisiert. Das Verfahrensprodukt fiel in Form farbloser Kristalle in 75 %iger Ausbeute an.
$^1$H-NMR-Spektrum (300 MHz, $d^6$-DMSO, Tetramethylsilan als Standard [0 ppm]):
0,68-1,2 ppm (m,4 H); 2,05-2,30 ppm (m, 1 H); 3,92 ppm (t, 2 H); 4,21 ppm (t, 2H); 6,58-7,42 ppm (m, 8 H); 10,38 ppm (s, 1 H).

Weitere physikalische Daten sind der nachstehenden Tabelle zu entnehmen, in der weitere Verbindungen

aufgeführt sind, die in analoger Weise hergestellt wurden oder herstellbar sind.

BASF Aktiengesellschaft

Tabelle 1

Neue Cyclopropanthiocarbonsäureamide

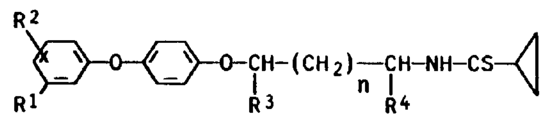

(I)

| Nr. | n | R1 | R2 | R3 | R4 | Fp. [°C] | IR-Daten [$cm^{-1}$] | Ausbeute [%] |
|---|---|---|---|---|---|---|---|---|
| 1 | 0 | F | H | H | H | 69 | 1507, 1487, 1248, 1240, 1209 1121, 962, 848, 841 | 75 |
| 2 | 0 | H | H | H | H | 60-62 | 1504, 1488, 1247, 1235, 1194, 994, 832 | 73 |
| 3 | 0 | H | 4-F | H | H | 65 | 1504, 1250, 1231, 1192, 1064, 994, 827 | 50 |
| 4 | 0 | Cl | H | H | H | 101 | 1505, 1472, 1464, 1216, 1192, 1056, 995, 967, 842 | 62 |
| 5 | 0 | Br | H | H | H | 56- 57 | 1505, 1489, 1468, 1229, 1222, 1058, 994 | 83 |
| 6 | 0 | Cl | 4-F | H | H | 99-100 | 1507, 1493, 1463, 1247, 1209, 1054, 998, 845, 784 | 68 |
| 7 | 0 | $CH_3$ | H | H | H | Oel | 1502, 1486, 1464, 1328, 1256, 1240, 1208, 1059, 996 | 81 |
| 8 | 0 | $CF_3$ | H | H | H | Oel | 1503, 1491, 1450, 1328, 1221, 1191, 1170, 1126, 1064, 914 | 90 |
| 9 | 0 | $C_2H_5$ | H | H | H | Oel | 1502, 1485, 1465, 1447, 1328, 1233, 1207, 1059, 996 | 87 |
| 10 | 0 | $OCH_3$ | H | H | H | | | |
| 11 | 0 | Cl | 4-Cl | H | H | | | |
| 12 | 0 | $OCF_3$ | H | H | H | | | |
| 13 | 0 | H | 4-Cl | H | H | | | |
| 14 | 0 | H | 4-Br | H | H | | | |
| 15 | 0 | $NO_2$ | H | H | H | | | |
| 16 | 0 | F | H | $CH_3$ | H | | | |
| 17 | 0 | H | H | $CH_3$ | H | | | |

BASF Aktiengesellschaft

Tabelle 1 (Forts.)

| Nr. | n | $R^1$ | $R^2$ | $R^3$ | $R^4$ | Fp. [°C] | IR-Daten [$cm^{-1}$] | Ausbeute [%] |
|---|---|---|---|---|---|---|---|---|
| 18 | 0 | H | 4-F | $CH_3$ | H | | | |
| 19 | 0 | Cl | H | $CH_3$ | H | | | |
| 20 | 0 | Br | H | $CH_3$ | H | | | |
| 21 | 0 | Cl | 4-F | $CH_3$ | H | | | |
| 22 | 0 | $CH_3$ | H | $CH_3$ | H | | | |
| 23 | 0 | $CF_3$ | H | $CH_3$ | H | | | |
| 24 | 0 | $C_2H_5$ | H | $CH_3$ | H | | | |
| 25 | 0 | $OCH_3$ | H | $CH_3$ | H | | | |
| 26 | 0 | Cl | 4-Cl | $CH_3$ | H | | | |
| 27 | 0 | $OCF_3$ | H | $CH_3$ | H | | | |
| 28 | 0 | H | 4-Cl | $CH_3$ | H | | | |
| 29 | 0 | H | 4-Br | $CH_3$ | H | | | |
| 30 | 0 | $NO_2$ | H | $CH_3$ | H | | | |
| 31 | 0 | F | H | H | $CH_3$ | | | |
| 32 | 0 | H | H | H | $CH_3$ | | | |
| 33 | 0 | H | 4-F | H | $CH_3$ | | | |
| 34 | 0 | Cl | H | H | $CH_3$ | | | |
| 35 | 0 | Br | H | H | $CH_3$ | | | |
| 36 | 0 | Cl | 4-F | H | $CH_3$ | | | |
| 37 | 0 | $CH_3$ | H | H | $CH_3$ | | | |
| 38 | 0 | $CF_3$ | H | H | $CH_3$ | | | |
| 39 | 0 | $C_2H_5$ | H | H | $CH_3$ | | | |

BASF Aktiengesellschaft

Tabelle 1 (Forts.)

| Nr. | n | $R^1$ | $R^2$ | $R^3$ | $R^4$ | Fp. [°C] | IR-Daten [$cm^{-1}$] | Ausbeute [%] |
|---|---|---|---|---|---|---|---|---|
| 40 | 0 | $OCH_3$ | H | H | $CH_3$ | | | |
| 41 | 0 | Cl | 4-Cl | H | $CH_3$ | | | |
| 42 | 0 | $OCF_3$ | H | H | $CH_3$ | | | |
| 43 | 0 | H | 4-Cl | H | $CH_3$ | | | |
| 44 | 0 | H | 4-Br | H | $CH_3$ | | | |
| 45 | 0 | $NO_2$ | H | H | $CH_3$ | | | |

Die Cyclopropanthiocarbonsäureamide sind geeignet, Schädlinge aus der Klasse der Insekten, Spinnentiere und Nematoden wirksam zu bekämpfen. Sie können im Pflanzenschutz sowie auf dem Hygiene-, Vorratsschutz- und Veterinärsektor als Schädlingsbekämpfungsmittel eingesetzt werden.

Zu den schädlichen Insekten gehören aus der Ordnung der Schmetterlinge (Lepidoptera) beispielsweise Agrotis ypsilon, Agrotis segetum, Alabama argillacea, Anticarsia gemmatalis, Argyresthia conjugella, Autographa gamma, Bupalus piniarius, Cacoecia murinana, Capua reticulana, Cheimatobia brumata, Choristoneura fumiferana, Choristoneura occidentalis, Cirphis unipuncta, Cydia pomonella, Dendrolimus pini, Diaphania nitidalis, Diatraea grndiosella, Earias insulana, Elasmopalpus lignosellus, Eupoecilia ambiguella, Evetria bouliana, Feltia subterranea, Galleria mellonella, Grapholita funebrana, Grapholita molesta, Heliothis armigera, Heliothis virescens, Heliothis zea, Hellula undalis, Hibernia defoliaria, Hyphantria cunea, Hyponomeuta malinellus, Keifferia lycopersicella, Lambdina fiscellaria, Laphygma exigua, Leucoptera coffeella, Leucoptera scitella, Lithocolletis blancardella, Lobesia botrana, Loxostege sticticalis, Lymantria dispar, Lymantria monacha, Lyonetia clerkella, Malacosoma neustria, Mamestra brassicae, Orgyia pseudotsugata, Ostrinia nubilalis, Panolis flamea, Pectinophora gossypiella, Peridroma saucia, Phalera bucephala, Phthorimaea operculella, Phyllocnistis citrella, Pieris brassicae, Plathypena scarbra, Plutella xylostella, Pseudoplusia includens, Phyacionia frustrana, Scrobipalpula absoluta, Sitotroga cerelella, Sparganothis pilleriana, Spodoptera frugiperda, Spodoptera littoralis, Spodoptera litura, Thaumatopoea pityocampa, Tortrix viridana, Trichoplusia ni, Zeiraphera canadensis.

Aus der Ordnung der Käfer (Coleoptera) beispielsweise Agrilus sinuatus, Agriotes lineatus, Agriotes obscurus, Amphimallus solstitialis, Anisandrus dispar, Anthonomus grandis, Anthonomus pomorum, Atomaria linearis, Blastophagus piniperda, Blitophaga undata, Bruchus rufimanus, Bruchus pisorum, Bruchus lentis, Byctiscus betulae, Cassida nebulosa, Cerotoma trifurcata, Ceuthorrhynchus assimilis, Ceuthorrynchus napi, Chaetocnema tibialis, Conoderus vespertinus, Crioceris asparagi, Diabrotica longicornis, Diabrotica 12-punctata, Diabrotica virgifera, Epilachna varivestis, Epitrix hirtipennis, Eutinobothrus brasiliensis, Hylobius abietis, Hypera brunneipennis, Hypera postica, Ips typographus, Lema bilineata, Lema melanopus, Leptinotarsa decemlineata, Limonius californicus, Lissorhoptrus oryzophilus, Melanotus communis, Meligethes aeneus, Melolontha hippocastani, Melolontha melolontha, Onlema oryzae, Ortiorrhynchus sulcatus, Otiorrhynchus ovatus, Phaedon cochleariae, Phyllotreta chrysocephala, Phyllophaga sp., Phyliopertha horticola, Phyllotreta nemorum, Phyllotreta striolata, Popillia japonica, Sitona lineatus, Sitophilus granaria.

Aus der Ordnung der Zweiflügler (Diptera) beispielsweise Aedes aegypti, Aedes vexans, Anastrepha ludens, Anopheles maculipennis, Ceratitis capitata, Chrysomya bezziana, Chrysomya hominivorax, Chrysomya macellaria, Contarinia sorghicola, Cordylobia anthropophaga, Culex pipiens, Dacus cucurbitae, Dacus oleae, Dasineura brassicae, Fannia canicularis, Gasterophilus intestinalis, Glossia morsitans, Haematobia irritans, Haplodiplosis equestris, Hylemyia platura, Hypoderma lineata, Liriomyza sativae, Liriomyza trifolii, Lucilia caprina, Lucilia cuprina, Lucilia sericata, Lycoria pectoralis, Mayetiola destructor, Musca domestica, Muscina stabulans, Oestrus ovis, Oscinella frit, Pegomya hysocyami, Phorbia antiqua, Phorbia brassicae, Phorbia coarctata, Rhagoletis cerasi, Rhagoletis pomonella, Tabanus bovinus, Tipula oleracea, Tipula paludosa.

Aus der Ordnung der Thripse (Thysanoptera) beispielsweise Frankliniella fusca, Frankliniella occidentalis, Frankliniella tritici, Scirtothrips citri, Thrips oryzae, Thrips palmi, Thrips tabaci.

Aus der Ordnung der Hautflügler (Hymenoptera) beispielsweise Athalia rosae, Atta cephalotes, Atta sexdens, Atta texana, Hoplocampa minuta, Hoplocampa testudinea, Monomorium pharaonis, Solenopsis geminata, Solenopsis invicta.

Aus der Ordnung der Wanzen (Heteroptera) beispielsweise Acrosternum hilare, Blissus leucopterus, Cyrtopeltis notatus, Dysdercus cingulatus, Dysdercus intermedius, Eurygaster integriceps, Euchistus impictiventris, Leptoglossus phyllopus, Lygus lineolaris, Lygus pratensis, Nezara viridula, Piesma quadrata, Solubea insularis, Thyanta perditor.

Aus der Ordnung der Pflanzensauger (Homoptera) beispielsweise Acyrthosiphon onobrychis, Adelges laricis, Aphidula nasturtii, Aphis fabae, Aphis pomi, Aphis sambuci, Brachycaudus cardui, Brevicoryne brassicae, Cerosipha gossypii, Dreyfusia nordmannianae, Dreyfusia piceae, Dyasphis radicola, Dysaulacorthum pseudosolani, Empoasca fabae, Macrosiphum avenae, Macrosiphum euphorbiae, Macrosiphon rosae, Megoura viciae, Metopolophium dirhodum, Myzodes persicae, Myzus cerasi, Nilaparvata lugens, Pemphigus bursarius, Perkinsiella saccharicida, Phorodon humuli, Psylla mali, Psylla piri, Rhopalomyzus ascalonicus, Rhopalosiphum maidis, Sappaphis mala, Sappaphis mali, Schizaphis graminum, Schizoneura lanuginosa, Trialeurodes vaporariorum, Viteus vitifolii.

Aus der Ordnung der Termiten (Isoptera) beispielsweise Calotermes flavicollis, Leucotermes flavipes, Reticulitermes lucifugus, Termes natalensis.

Aus der Ordnung der Geradflügler (Orthoptera) beispielsweise Acheta domestica, Blatta orientalis, Blattella germanica, Forficula auricularia, Gryllotalpa gryllotalpa, Locusta migratoria, Melanoplus birittatus,

Melanoplus femur-rubrum, Melanoplus mexicanus, Melanoplus sanguinipes, Melanoplus spretus, Nomadacris septemfasciata, Periplaneta americana, Schistocerca americana, Schistocerca peregrina, Stauronotus maroccanus, Tachycines asynamorus.

Zur Klasse der Arachnoidea gehören Spinnentiere (Acarina) beispielsweise Amblyomma americanum, Amglyomma variegatum, Argas persicus, Boophilus annulatus, Boophilus decoloratus, Boophilus microplus, Brevipalpus phoenicis, Bryobia praetiosa, Dermacentor silvarum, Eotetranychus carpini, Eriophyes sheldoni, Hyalomma truncatum, Ixodes ricinus, Ixodes rubicundus, Ornithodorus moubata, Otobins megnini, Paratetranychus pilosus, Permanyssus gallinae, Phyllocaptrata oleivora, Polyphagotarsonemus latus, Psoroptes ovis, Rhipicephalus appendiculatus, Rhipicephalus evertsi, Saccoptes scabiei, Tetranychus cinnabarinus, Tetranychus kanzawai, Tetranychus pacificus, Tetranychus telarius, Tetranychus urticae.

Zur Klasse der Nematoden zählen beispielsweise Wurzelgallennematoden, z.B. Meloidogyne hapla, Meloidogyne incognita, Meloidogyne javanica, Zysten bildende Nematoden, z.B. Globodera rostochiensis, Heterodera avenae, Hetrodera glycinae, Heterodera schatii, Hetrodera triflolii, Stock- und Blattälchen, z.B. Belonolaimus longicaudatus, Ditylenchus destructor, Ditylenchus dipsaci, Heliocotylenchus multicinctus, Longidorus elongatus, Radopholus similis, Rotylenchus robustus, Trichodorus primitivus, Tylenchorhynchus claytoni, Tylenchorhynchus dubius, Pratylenchus neglectus, Pratylenchus penetrans, Paratylenchus curvitatus, Partylenchus goodeyi.

Die Wirkstoffe können als solche, in Form ihrer Formulierungen oder den daraus bereiteten Anwendungsformen, z.B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate, Methanol, Ethanol, Propanol, Butanol, Chloroform, Tetrachlorkohlenstoff, Cyclohexanol, Cyclohexanon, Chlorbenzol, Isophoron, stark polare Lösungsmittel, z.B. Dimethylformamid, Dimethylsulfoxid, N-Methylpyrrolidon, Wasser, in Betracht.

Wäßrige Anwendungsformen können aus Emulsionskonzentraten, Pasten oder netzbaren Pulvern (Spritzpulver, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermitttel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphthalinsulfonsäure mit Phenol und Formaldehyd, Polyoxyethylenoctylphenolether, ethoxyliertes Isooctylphenol, Octylphenol, Nonylphenol, Alkylphenolpolyglykolether. Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin-Sulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Beispiele für Formulierungen sind:

I. 5 Gew.-Teile der Verbindung Nr. 1 werden mit 95 Gew.-Teilen feinteiligem Kaolin innig vermischt. Man erhält auf diese Weise ein Stäubemittel, das 3 Gew.-% des Wirkstoffs enthält.
II. 30 Gew.-Teile der Verbindung Nr. 2 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.
III. 10 Gew.-Teile der Verbindung Nr. 3 werden in einer Mischung gelöst, die aus 90 Gew.-Teilen Xylol, 6

Gew.-Teilen des Anlagerungsproduktes von 8 bis 10 Mol Ethylenoxid an 1 Mol Ölsäure-N-monoethanolamid, 2 Gew.-Teilen Calciumsalz der Dodecylbenzolsulfonsäure und 2 Gew.-Teilen des Anlagerungsproduktes von 40 Mol Ethylenoxid an 1 Mol Ricinusöl besteht.

IV. 20 Gew.-Teile der Verbindung Nr. 4 werden in einer Mischung gelöst, die aus 60 Gew.-Teilen Cyclohexanon, 30 Gew.-Teilen Isobutanol, 5 Gew.-Teilen des Anlagerungsproduktes von 7 Mol Ethylenoxid an 1 Mol Isooctylphenol und 5 Gew.-Teilen des Anlagerungsproduktes von 40 Mol Ethylenoxid an 1 Mol Ricinusöl besteht.

V. 80 Gew.-Teile der Verbindung Nr. 5 werden mit 3 Gew.-Teilen des Natriumsalzes der Diisobutylnaphthalin-alpha-sulfonsäure, 10 Gew.-Teilen des Natriumsalzes einer Ligninsulfonsäure aus einer Sulfit-Ablauge und 7 Gew.-Teilen pulverförmigem Kieselsäuregel gut vermischt und in einer Hammermühle vermahlen.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden, wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die Formulierungen enthalten im allgemeinen zwischen 0,1 und 95 Gew.% Wirkstoff, vorzugsweise zwischen 0,5 und 90 Gew.%.

Die Wirkstoffkonzentrationen in den anwendungsfertigen Zubereitungen können in größeren Bereichen variiert werden.

Im allgemeinen liegen sie zwischen 0,0001 und 10 %, vorzugsweise zwischen 0,01 und 1 %.

Die Wirkstoffe können auch mit gutem Erfolg im Ultra-Low-Volume-Verfahren (ULV) verwendet werden, wobei es möglich ist, Formulierungen mit mehr als 95 Gew.% Wirkstoff oder sogar den Wirkstoff ohne Zusätze auszubringen.

Die Aufwandmenge an Wirkstoff beträgt unter Freilandbedingungen 0,02 bis 10, vorzugsweise 0,1 bis 2,0 kg/ha.

Zu den Wirkstoffen können Öle verschiedenen Typs, Herbizide, Fungizide, andere Schädlingsbekämpfungsmittel, Bakterizide, gegebenenfalls auch erst unmittelbar vor der Anwendung (Tankmix), zugesetzt werden. Diese Mittel können zu den erfindungsgemäßen Mitteln im Gewichtsverhältnis 1:10 bis 10:1 zugemischt werden.

Anwendungsbeispiele

Die Konzentrationen, bei denen die untersuchten Verbindungen eine 100 %ige bzw. 80 %ige Mortalität bzw. Hemmung bewirken, sind die jeweiligen Minimalkonzentrationen (Wirkschwelle).

Die Reinheit der Substanzen lag bei > 95 %.

Folgende Formulierungen wurden verwendet:

a) - eine 0,1 %ige Lösung des Wirkstoffes in Aceton, die entsprechend den angegebenen Dosierungen weiter mit Aceton verdünnt wurde;

b) - eine 10 %ige Emulsion des Wirkstoffs in einem Gemisch aus 70 Gew.-% Cyclohexanol, 20 Gew.-% Nekanil LN® (≙ Lutensol AP6, Netzmittel mit Emulgier- und Dispergierwirkung auf Basis ethoxylierter Alkylphenole) und 10 Gew.-% Emulphor EL® (≙ Emulan EL®, Emulgator auf Basis ethoxylierter Fettalkohole), die mit Wasser auf die in den Beispielen angegebenen Konzentrationen verdünnt wurde.

Beispiel A

Dysdercus intermedius (Baumwollwanze); ovizide Wirkung

Stecketiketten wurden am oberen Rand mit Klebstreifenstückchen (ca. 0,8 cm) beklebt. 24 Stunden vor Versuchsbeginn wurden die Eier durch Eintauchen in das "Sammelgefäß" an dem Klebstreifenrand befestigt. Die Eier wurden für ca. 5 Sekunden in die wäßrige Wirkstoffaufbereitung getaucht, anschließend ließ man auf Filterpapier abtropfen. Dabei sollen die Eier nicht auf das Papier gelegt werden.

Die zurechtgeschnittenen Etiketten wurden mit den Klebstreifen nach oben in Gefäße gestellt, die zur Vermeidung von Austrocknung jeweils eine mit Wasser durchfeuchtete, halbe Watterolle enthielten. Nach Abdecken der Gefäße mit Glasplatten wurde gewartet, bis die Larven in einem Kontrollexperiment (ohne Wirkstoff) geschlüpft waren (ca. 8 Tage) und dann bonitiert.

Bei einer Wirkstoffkonzentration von 0,1 bis 10 ppm bewirkten die Verbindungen 1, 3, 4, 5, 6 und 7 eine

Mortalität von 80 bis 100 %.

Beispiel B

Dysdercus intermedius (Baumwollwanze) ; Zuchtversuch

Je 200 g steriler Quarzsand wurden mit 25 ml wäßriger Wirkstoffaufbereitung vermischt, in 1 l Glasgefäße gegeben und mit ca. 20 Larven von Dysdercus im 3. Larvenstadium besetzt. Der Sand wurde 1 mal wöchentlich angefeuchtet und die Larven mit eingeweichter Baumwollsaat gefüttert. Der Versuch lief bis zum Schlüpfen der Folgegeneration. Dann wurde die Wirkung in % Mortalität ermittelt.

Bei einer Wirkstoffkonzentraton von 0,01 bis 1 ppm bewirkten die Verbindungen 1 bis 7 und 9 eine Mortalität von 100 %.

Beispiel C

Musca domestica (Stubenfliegen); Zuchtversuch

25 ml einer trockenen Futtermischung aus 1 kg Kleie, 250 g Hefepulver und 35 g Fischmehl wurden in 100 ml Becher gefüllt und der Wirkstoff mit 25 ml einer Milch-Zucker-Lösung (42 $cm^3$ Zucker auf 1 l Milch) zugemischt. Danach wurden in jeden Becher 20 Larven im 1. Larvenstadium (L 1) plaziert und die Becher mit gelochten Deckeln verschlossen. Der Versuch lief bis zum Schlüpfen der Fliegen in einem Kontrollexperiment (ohne Wirkstoff).

Bei einer Wirkstoffkonzentration von 20 bis 40 ppm bewirkten die Verbindungen 1, 3, 4, 5, 6, 7 und 9 eine Mortalität von 100 %.

Beispiel D

Prodenia litura (Ägypt. Baumwollwurm); Zuchtversuch auf behandeltem Nährboden

Glaspetrischalen (∅ 10 cm) wurden mit der acetonischen Wirkstoffaufbereitung [a)] behandelt, nach dem Verdunsten des Lösungsmittels 5 Raupen im 4. Larvenstadium eingesetzt und die Schalen verschlossen. Nach 4 Stunden wurde die Mortalität festgestellt und jeweils 5 der überlebenden Raupen (Länge 10 - 12 mm) in einen 250 ml Becher plaziert. Diese Becher enthielten ca. 100 ml eines Standard-Nährbodens (3,1 l Wasser, 80g Agar, 137 g Bierhefe, 515 g Maismehl, 130 g Weizenkeime und übliche Zusatzstoffe und Vitamine), dem im flüssigen Zustand der Wirkstoff sorgfältig untergemischt worden war und perforierte, transparente Deckel. Der Beobachtungszeitraum erstreckte sich bis zum Schlüpfen der Falter in einem Kontrollexperiment (ohne Wirkstoff).

Bei einer Wirkstoffkonzentration von 0,002 bis 0,004 ppm bewirkten die Verbindungen 1 und 3 bis 9 eine Mortalität von 80 bis 100 %.

## Patentansprüche

### Patentansprüche für folgende Vertragsstaaten: AT, BE, CH, DE, FR, GB, IT, LI, NL, SE

1. Cyclopropanthiocarbonsäureamide der allgemeinen Formel I

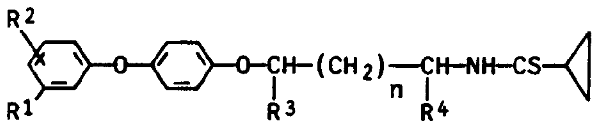

(I)

in der die Variablen folgende Bedeutung haben:

| | |
|---|---|
| $R^1$, $R^2$ | Wasserstoff, Cyano, Nitro, Halogen, $C_1$-$C_4$-Alkyl oder $C_1$-$C_4$-Alkoxy, wobei die Alkyl- und Alkoxyreste partiell oder vollständig halogeniert sein können; |
| $R^3$, $R^4$ | Wasserstoff oder $C_1$-$C_4$-Alkyl; |
| n | 0 oder 1. |

2. Cyclopropanthiocarbonsäureamide der Formel I nach Anspruch 1, in denen $R^3$ und $R^4$ für Wasserstoff stehen und n den Wert null hat.

3. Verfahren zur Herstellung der Cyclopropanthiocarbonsäureamide I gemäß Anspruch 1, dadurch gekennzeichnet, daß man ein 4-Phenoxyphenoxyalkylamin der allgemeinen Formel II

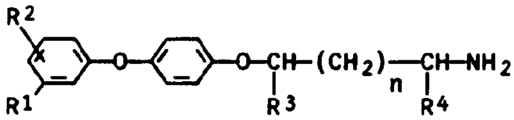

(II)

mit einem Cyclopropandithiocarbonsäureester der allgemeinen Formel III

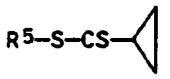

(III),

in der $R^5$ ein kurzkettiges Alkyl bedeutet,
umsetzt.

4. Verwendung der Cyclopropanthiocarbonsäureamide I gemäß Anspruch 1 zur Bekämpfung von Schädlingen.

5. Schädlingsbekämpfungsmittel, enthaltend mindestens ein Cyclopropancarbonsäurethioamid der Formel I gemäß Anspruch 1 und inerte Trägerstoffe.

6. Schädlingsbekämpfungsmittel nach Anspruch 5, enthaltend 0,1 bis 99 Gew.% eines Cyclopropanthiocarbonsäureamids der Formel I.

7. Verfahren zur Bekämpfung von Schädlingen, dadurch gekennzeichnet, daß man eine wirksame Menge eines Cyclopropanthiocarbonsäureamids der Formel I gemäß Anspruch 1 auf Schädlinge bzw. ihren Lebensraum einwirken läßt.

**Patentansprüche für fogenden Vertragsstaat: ES**

1. Verfahren zur Herstellung von Cyclopropanthiocarbonsäureamiden der allgemeinen Formel I

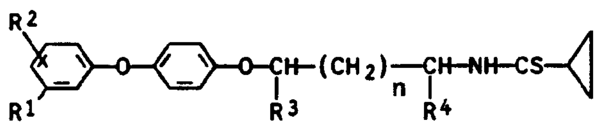

I,

in der die Variablen folgende Bedeutung haben:

| | |
|---|---|
| $R^1$, $R^2$ | Wasserstoff, Cyano, Nitro, Halogen, $C_1$-$C_4$-Alkyl oder $C_1$-$C_4$-Alkoxy, wobei die Alkyl- und Alkoxyreste partiell oder vollständig halogeniert sein können; |
| $R^3$, $R^4$ | Wasserstoff oder $C_1$-$C_4$-Alkyl; |
| n | 0 oder 1 |

dadurch gekennzeichnet, daß man ein 4-Phenoxyphenoxy-alkylamin der allgemeinen Formel II

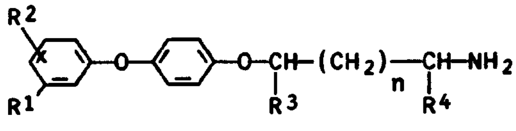

II

mit einem Cyclopropandithiocarbonsäureester der allgemeinen Formel III

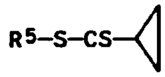

III

in der $R^5$ ein kurzkettiges Alkyl bedeutet, umsetzt.

2. Verfahren nach Anspruch 1, dadurch gekennzeichnet, daß man es auf die Herstellung von solchen Cyclopropanthiocarbonsäureamiden I anwendet, bei denen die Substituenten $R^3$ und $R^4$ für Wasserstoff stehen und n den Wert null hat.

3. Verwendung der Cyclopropanthiocarbonsäureamide I gemäß Anspruch 1 zur Bekämpfung von Schädlingen.

4. Schädlingsbekämpfungsmittel, enthaltend mindestens ein Cyclopropancarbonsäurethioamid der Formel I gemäß Anspruch 1 und inerte Trägerstoffe.

5. Schädlingsbekämpfungsmittel nach Anspruch 4, enthaltend 0,1 bis 99 Gew.% eines Cyclopropanthiocarbonsäureamids der Formel I.

6. Verfahren zur Bekämpfung von Schädlingen, dadurch gekennzeichnet, daß man eine wirksame Menge eines Cyclopropanthiocarbonsäureamids der Formel I gemäß Anspruch 1 auf Schädlinge bzw. ihren Lebensraum einwirken läßt.

## Claims

### Claims for the following Contracting States: AT, BE, CH, DE, FR, GB, IT, LI, NL, SE

1. A cyclopropanethiocarboxamide of the general formula I

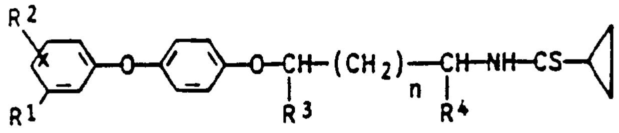

(I),

where $R^1$ and $R^2$ are hydrogen, cyano, nitro, halogen, $C_1$-$C_4$-alkyl or $C_1$-$C_4$-alkoxy, it being possible for the alkyl and alkoxy radicals to be partially or completely halogenated, $R^3$ and $R^4$ are hydrogen or $C_1$-$C_4$-alkyl and n is 0 or 1.

2. A cyclopropanethiocarboxamide of the formula I as claimed in claim 1, where $R^3$ and $R^4$ are hydrogen and n is zero.

3. A process for the preparation of a cyclopropanethiocarboxamide I as claimed in claim 1, wherein a 4-phenoxyphenoxyalkylamine of the general formula II

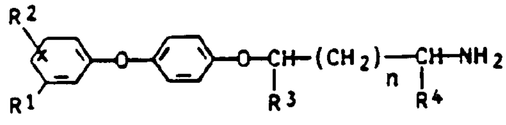

(II)

is reacted with a cyclopropanedithiocarboxylate of the general formula III

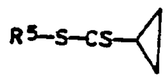

(III),

where $R^5$ is short-chain alkyl.

4. Use of a cyclopropanethiocarboxamide I as claimed in claim 1 for controlling pests.

5. A pesticide containing at least one cyclopropane- thiocarboxamide of the formula I as claimed in claim 1 and inert carriers.

6. A pesticide as claimed in claim 5, containing from 0.1 to 99 % by weight of a cyclopropanethiocarboxamide of the formula I.

7. A method for controlling pests, wherein an effective amount of a cyclopropanethiocarboxamide of the formula I as claimed in claim 1 is allowed to act on pests or their habitat.

**Claims for the following Contracting State: ES**

1. A process for preparing cyclopropanethiocarboxamides of the general formula I

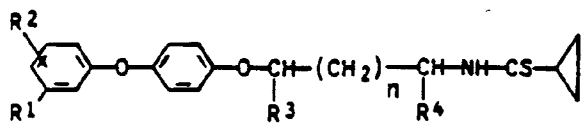

I,

where $R^1$ and $R^2$ are hydrogen, cyano, nitro, halogen, $C_1$-$C_4$-alkyl or $C_1$-$C_4$-alkoxy, it being possible for the alkyl and alkoxy radicals to be partially or completely halogenated, $R^3$ and $R^4$ are hydrogen or $C_1$-$C_4$-alkyl and n is 0 or 1, wherein a 4-phenoxyphenoxyalkylamine of the general formula II

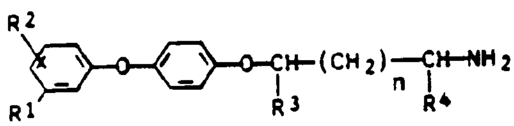

II

is reacted with a cyclopropanedithiocarboxylate of the general formula III

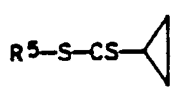

III

where $R^5$ is short-chain alkyl.

2. A process as claimed in claim 1, which is used for the preparation of a cyclopropanecarboxamide I in which $R^3$ and $R^4$ are each hydrogen and n is zero.

3. Use of a cyclopropanethiocarboxamide I as claimed in claim 1 for controlling pests.

4. Pesticides containing at least one cyclopropanethiocarboxamide of the formula I as claimed in claim 1 and inert carriers.

5. A pesticide as claimed in claim 4, containing from 0.1 to 99 % by weight of a cyclopropanethiocarboxamide of the formula I.

6. A method for controlling pests, wherein an effective amount of a cyclopropanethiocarboxamide of the formula I as claimed in claim 1 is allowed to act on pests or their habitat.

## Revendications

### Revendications pour les Etats contractants suivants: AT, BE, CH, DE, FR, GB, IT, LI, NL, SE

1. Cyclopropane-thiocarboxamides de formule générale I

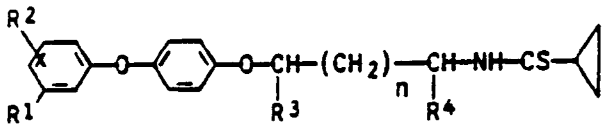

(I)

dans laquelle les divers symboles ont les significations suivantes :
$R^1$, $R^2$ représentent l'hydrogène, des groupes cyano, nitro, des halogènes, des groupes alkyle en C1-C4 ou alcoxy en C1-C4, les groupes alkyle et alcoxy pouvant être halogénés en totalité ou en partie ;
$R^3$, $R^4$ représentent l'hydrogène ou des groupes alkyle en C1-C4 ;
n est égal à 0 ou 1.

2. Cyclopropane-thiocarboxamides de formule I de la revendication 1 dans laquelle $R^3$ et $R^4$ représentent l'hydrogène et n est égal à 0.

3. Procédé de préparation des cyclopropane- thiocarboxamides I selon la revendication 1 caractérisé en ce que l'on fait réagir une 4- phénoxyphénoxyalkylamine de formule générale II

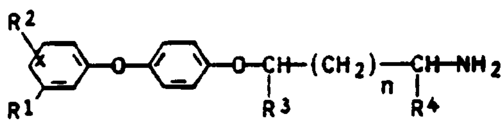

(II)

avec un ester d'acide cyclopropane-dithiocarboxylique de formule générale III

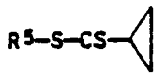

(III),

dans laquelle $R^5$ représente un groupe alkyle à chaîne courte.

4. Utilisation des cyclopropane-thiocarboxamides I de la revendication 1 pour la lutte contre les parasites.

5. Produit parasiticide, contenant au moins un thioamide d'acide cyclopropane-carboxylique de formule I de la revendication 1 et des véhicules inertes.

6. Produit parasiticide selon la revendication 5, contenant de 0,1 à 99 % en poids d'un cyclopropane- thiocarboxamide de formule I.

7. Procédé pour combattre les parasites, caractérisé en ce que l'on fait agir une quantité efficace d'un cyclopropane-thiocarboxamide de formule I de la revendication 1 sur les parasites ou leur habitat.

### Revendications pour l'Etat contractant suivant: ES

1. Procédé de préparation des cyclopropanethiocarboxamides de formule générale I

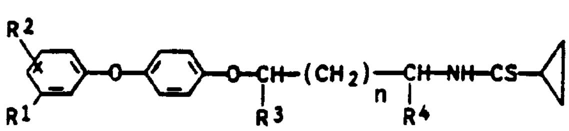

I,

dans laquelle les divers symboles ont les significations suivantes :
$R^1$, $R^2$ représentent l'hydrogène, des groupes cyano, nitro, des halogènes, des groupes alkyle en C1-C4 ou alcoxy en C1-C4, les groupes alkyle et alcoxy pouvant être halogénés en totalité ou en partie ;
$R^3$, $R^4$ représentent l'hydrogène ou des groupes alkyle en C1-C4 ; n est égal à 0 ou 1, caractérisé en ce que l'on fait réagir une 4-phénoxyphénoxy-alkylamine de formule générale II

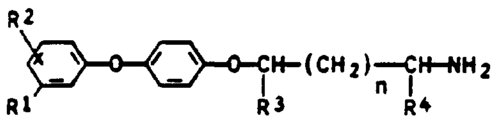

II

avec un ester d'acide cyclopropane-dithiocarboxylique de formule générale III

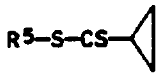

III

dans laquelle $R^5$ représente un groupe alkyle à chaîne courte.

2. Procédé selon la revendication 1, caractérisé en ce que l'on prépare des cyclopropane-thiocarboxamides I dans lesquels les symboles $R^3$ et $R^4$ représentent l'hydrogène et n est égal à 0.

3. Utilisation des cyclopropane-thiocarboxamides I de la revendication 1 pour la lutte contre les parasites.

4. Produit parasiticide, contenant au moins un thioamide d'acide cyclopropane-carboxylique de formule I de la revendication 1 et des véhicules inertes.

5. Produit parasiticide selon la revendication 4, contenant de 0,1 à 99 % en poids d'un cyclopropane- thiocarboxamide de formule I.

6. Procédé pour combattre les parasites, caractérisé en ce que l'on fait agir une quantité efficace d'un cyclopropane-thiocarboxamide de formule I de la revendication 1 sur les parasites ou leur habitat.